# FASCICULE DE BREVET EUROPEEN

(11) **EP 1 477 128 B1**
(45) Date de publication et mention de la délivrance du brevet: **18.02.2009**
(21) Numéro de dépôt: 04076445.8
(22) Date de dépôt: 14.05.2004
(51) Int. Cl.: A61D 1/02

(54) **Ensemble comprenant un capuchon et une seringue d'injection intra-mammaire**
Baugruppe bestehend aus einer Verschlusskappe und einer intramammären Injektionsspritze
Assembly comprising a cap and an intramammary injection syringe

(30) Priorité: 16.05.2003 EP 03076484
(43) Date de publication de la demande: 17.11.2004
(73) Titulaire: Hubert De Backer NV, 9100 Sint Niklaas (BE)
(72) Inventeur: De Backer, Jan, 9100 Sint-Niklaas (BE)
(74) Mandataire: Quintelier, Claude

(56) Documents cités:
- EP-A- 0 540 493
- EP-A- 0 876 824
- DE-A- 19 915 272

## Description

La présente invention se rapporte à un ensemble comprenant un capuchon et une seringue d'injection intra-mammaire pourvue d'une canule et d'un réservoir et agencée pour injecter une substance médicamenteuse dans le canal d'une tétine du pis d'un animal, laquelle canule et le capuchon étant fabriqués en une matière élastique lequel capuchon comprend une partie inférieure agencée pour être fixée à la base de la canule à hauteur où la canule est fixée au réservoir, lequel capuchon comprend également une partie supérieure rendue solidaire de la partie inférieure par une liaison et agencée pour recouvrir la partie supérieure de la canule, les parties inférieure et supérieure du capuchon étant agencées pour que lorsqu'il est fixé à la canule, la partie supérieure du capuchon est détachable de sa partie inférieure par un mouvement exercé sur la partie supérieure du capuchon sans que la partie inférieure ne soit ainsi détachée de la canule. ladite liaison entre la partie inférieure et la partie supérieure du capuchon étant agencée pour être rompue sous l'effet dudit mouvement formé par application d'une flexion causant une inclinaison latérale de ladite partie supérieure.

Un tel ensemble est connu de la demande de brevet EP-A-0540493. Dans cet ensemble, le capuchon comprend une partie inférieure et une partie supérieure. La partie inférieure du capuchon est ancrée sur le réservoir à partir duquel s'étend la canule. Cette dernière est formée par une canule. La partie supérieure du capuchon vient à son tour envelopper la canule pour la protéger. Les parties inférieures et supérieures du capuchon sont rendues solidaires par une liaison dans la masse même du capuchon. Cette liaison forme également une entaille dans le capuchon de telle façon à former une ligne de rupture. Pour détacher la partie supérieure de la partie inférieure du capuchon il faut exercer un mouvement sur la partie supérieure pour briser ainsi le capuchon à hauteur de la ligne de rupture. Le fait que la partie supérieure puisse ainsi être désolidarisée de la partie inférieure du capuchon, permet de vérifier si l'ensemble a été utilisé. En effet, après avoir détaché la partie supérieure de la partie inférieure, cette dernière reste fixée sur le réservoir et l'utilisateur pourra ainsi tout de suite constater que l'ensemble a été ouvert et donc utilisé. Le fait que la canule et le capuchon sont fabriqués en matière élastique permet d'exercer une flexion tant sur la canule que sur le capuchon sans pour autant détruire le caractère essentiellement cylindrique de la canule. La matière élastique de la canule veillera que celle-ci, même après flexion, reprenne son caractère essentiellement cylindrique.

Le document DOS 199 15 272 décrit également un ensemble formé d'un capuchon et d'une seringue d'injection. La seringue est formée par une aiguille, en général en acier, qu'il faut protéger à l'aide du capuchon. L'usage de ce type d'aiguille impose d'exercer un mouvement de rotation sur le capuchon pour le détacher de la partie inférieure.

Un inconvénient du mouvement de rotation décrit dans DOS 199 15 272 est qu'il impose de tenir l'ensemble dans une main et d'exercer la rotation avec l'autre main. Ceci est d'autant plus gênant si l'utilisateur désire garder une main libre soit pour tenir un chiffon ou pour prendre en main la mammaire de l'animal.

L'invention a pour but de réaliser un ensemble comprenant un capuchon et une seringue d'injection où il est possible de détacher la partie supérieure du capuchon en n'utilisant qu'une seule main.

A cette fin, un ensemble suivant l'invention est caractérisé en ce que la partie inférieure du capuchon a une longueur, considérée dans sa direction axiale, qui est inférieure à celle de la partie supérieure. L'application d'une flexion causant l'inclinaison latérale de la partie supérieure va permettre la rupture entre la partie supérieure et inférieure à hauteur de la liaison. Puisqu'il suffit d'appliquer une flexion pour causer cette rupture, cette flexion peut être appliquée par le pouce en tenant l'ensemble dans une main. Ainsi l'autre main reste libre.

Une première forme de réalisation préférentielle d'un ensemble suivant l'invention est caractérisée en ce que la longueur de ladite partie inférieure du capuchon est inférieure ou égale à 1.5 fois, de préférence 1 fois la plus petite dimension de sa base, mesurée orthogonalement à ladite longueur. Ceci impose d'exercer la flexion sur la partie supérieure car la longueur de la partie inférieure est trop courte pour exercer une pression suffisante. Pour une même raison la longueur de ladite partie inférieure du capuchon est inférieure ou égale à 1 cm.

Une deuxième forme de réalisation préférentielle d'un ensemble suivant l'invention est caractérisée en ce que les parties inférieure et supérieure du capuchon font corps l'une avec l'autre et en ce qu'une entaille périphérique est agencée entre elles pour permettre de détacher la partie supérieure de l'inférieure par l'application de ladite flexion sur la face latérale de ladite partie supérieure. L'usage d'une entaille périphérique facilite la formation de la ligne de rupture entre la partie inférieure et supérieure du capuchon.

De préférence, le sommet de la partie inférieure dudit capuchon présente, une fois la partie supérieure du capuchon détachée de sa partie inférieure, un rebord agencé pour définir avec la face latérale de la canule un canal périphérique pour récolter d'éventuelles impuretés présentes sur une tétine une fois ladite canule introduite dans cette tétine.

L'invention va à présent être décrite plus en détails sur base d'un exemple de réalisation non limitatif de la portée de cette invention et en référence aux dessins dans lesquels :
la figure 1 montre une vue en coupe de l'ensemble suivant l'invention; et
les figures 2 a à 2 d illustrent l'ouverture de l'ensemble.

Dans les dessins un même signe de référence a été attribué à un même élément ou à un élément analogue.

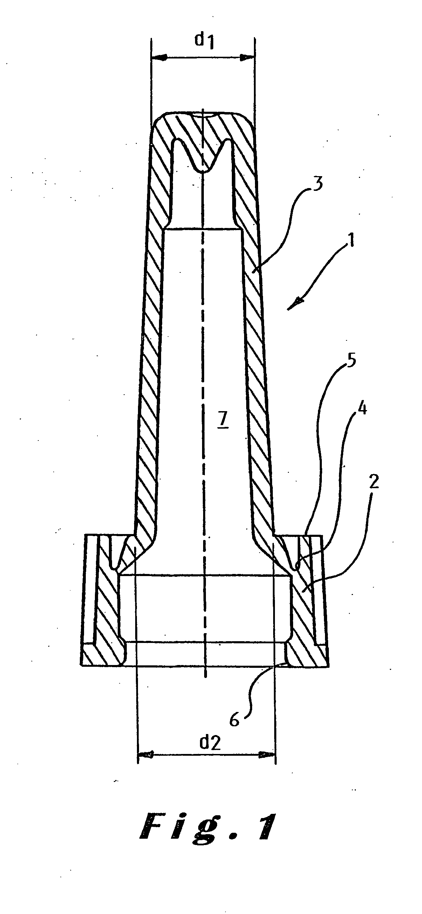

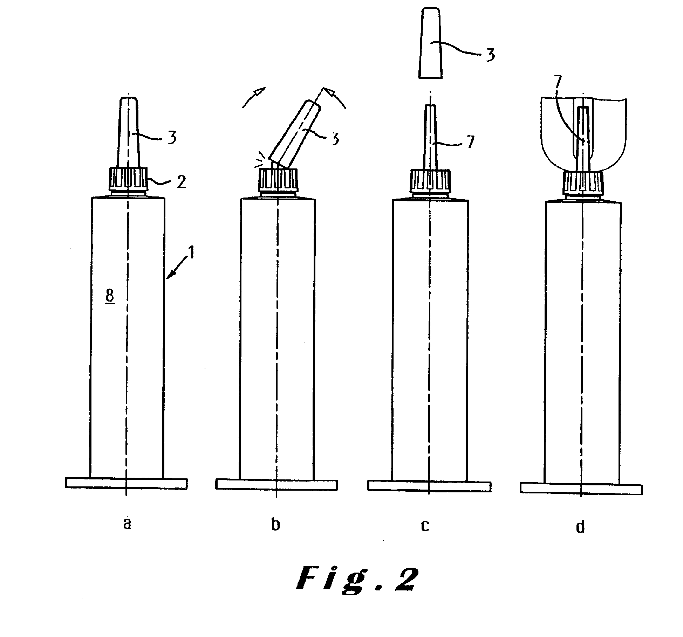

La figure 1 représente un capuchon 1 faisant partie d'un ensemble selon l'invention. Le capuchon recouvre une seringue d'injection intra-mammaire pourvue d'une canule 7 et d'un réservoir 8 (voir figure 2). L'ensemble est agencé pour injecter une substance médicamenteuse dans le canal d'une tétine du pis d'un animal. Le capuchon 1 comprend une partie inférieure 2 agencée pour être fixée à la base de la canule au moyen d'une lèvre périphérique 6 agencée pour coopérer par forçage avec une gorge correspondante agencée à la base de la canule et une partie supérieure 3 agencée pour recouvrir la partie supérieure de la canule. La partie inférieure est agencée pour être fixée à la base de la canule à hauteur où la canule rejoint le réservoir.

Le parties inférieure 2 et supérieure 3 du capuchon 1 sont agencées pour que lorsqu'il est fixé à la canule, la partie supérieure 3 du capuchon 1 peut être détachée de sa partie inférieure 2 par application d'une flexion causant une inclinaison latérale de la partie supérieure. La pression est par exemple appliquée à l'aide du pouce sur la face latérale de ladite partie supérieure 3, sans que la partie inférieure 2 ne soit ainsi détachée de la canule. Ainsi, la partie inférieure 2 du capuchon 1 demeure fixée à la base de la canule, une fois la partie supérieure 3 dudit capuchon 1 retirée. Les parties inférieure 2 et supérieure 3 du capuchon sont rendues solidaires entre elles par une liaison formant une entaille périphérique 4. Lors de l'application d'une flexion (figure 2 b) sur la face latérale de la partie supérieure 3 du capuchon en vue de la séparer de sa partie inférieure 2, l'entaille prévue entre elles permet de rompre le lien qui les unit et ainsi d'obtenir leur séparation (figure 2 c).

Il est essentiel selon l'invention, que la pression appliquée en vue de séparer la partie supérieure 3 de la partie inférieure 2 du capuchon 1 soit insuffisante pour séparer la partie inférieure 2 de la canule. Ainsi, cette partie inférieure 2 du capuchon 1 demeure fixée à la canule une fois la partie supérieure 3 détachée de cette partie inférieure 2, de sorte à constituer un élément témoin de l'utilisation de la canule. En effet, la partie supérieure 3 du capuchon 1 n'est en pratique séparée de la partie inférieure 2 de ce capuchon que préalablement à l'utilisation de la canule.

La longueur de la partie inférieure 2 du capuchon 1, considérée dans sa direction axiale, est choisie de sorte que cette partie inférieure 2 ne puisse pas être détachée de la canule par application d'une pression digitale sur la face latérale de ladite partie inférieure 2. Pour obtenir ce résultat, la longueur de la partie inférieure 2 du capuchon 1 est choisie inférieure à la plus petite dimension de la base de cette partie, mesurée orthogonalement à ladite longueur, à savoir dans le cas d'un capuchon 1 de section transversale généralement circulaire, au diamètre de la base de la partie inférieure 2 de ce capuchon 1. Typiquement, la longueur de ladite partie inférieure 2 du capuchon 1, est inférieure ou égale à 1 cm, cette dimension correspondant au diamètre maximal généralement utilisé pour la base d'une partie inférieure 2 du capuchon 1 selon l'invention.

Le sommet de la partie inférieure 2 du capuchon 1 présente un rebord 5 agencé pour, une fois la partie supérieure 3 du capuchon détachée de sa partie inférieure 2, définir avec la face latérale de la canule un canal périphérique pour récolter d'éventuelles impuretés présente sur une tétine de l'animal traité au moyen de la canule portant la partie inférieure 2 du capuchon 1 selon l'invention, une fois cette canule introduite dans le canal de la tétine en question.

Pour permettre cette inclinaison latérale, la canule et le capuchon sont fabriqués en une matière élastique comme par exemple le PET. Ainsi le caractère essentiellement cylindrique de la canule et aussi du capuchon n'est pas endommagé par la flexion. La matière élastique permet en effet à la canule de reprendre sa forme d'origine après flexion et donc de ne pas perturber le passage de la substance médicamentaire.

De préférence le diamètre d1 mesuré au niveau supérieur du capuchon 3 et le diamètre d2 mesuré au niveau inférieur du capuchon respectent la relation d2 ≥ d1. Ceci permet de fabriquer le capuchon 3 et la canule par un procédé de moulage et de retirer facilement le capuchon et la canule du moule.

## Revendications

1. Ensemble comprenant un capuchon (1) et une seringue d'injection intra-mammaire pourvue d'une canule et d'un réservoir et agencée pour injecter une substance médicamenteuse dans le canal d'une tétine du pis d'un animal, laquelle canule et le capuchon étant fabriqués en une matière élastique, lequel capuchon comprend une partie inférieure (2) agencée pour être fixée à la base de la canule à hauteur où la canule est fixée au réservoir, lequel capuchon comprend également une partie supérieure (3) rendue solidaire de la partie inférieure (2) par une liaison et agencée pour recouvrir la partie supérieure de la canule, les parties inférieure (2) et supérieure (3) du capuchon (1) étant agencées pour que lorsqu'il est fixé à la canule, la partie supérieure (3) du capuchon est détachable de sa partie inférieure (2) par un mouvement exercé sur la partie supérieure du capuchon sans que la partie inférieure (2) ne soit ainsi détachée de la canule, ladite liaison entre la partie inférieure et la partie supérieure du capuchon étant agencée pour être rompue sous l'effet dudit mouvement formé par application d'une flexion causant une inclinaison latérale de ladite partie supérieure (3) **caractérisé en ce que** la partie inférieure (2) du capuchon a une longueur, considérée dans sa direction axiale, qui est inférieure à celle de la partie supérieure (3).

2. Ensemble selon la revendication 1, **caractérisé en ce que** la longueur de ladite partie inférieure (2) du capuchon est inférieure ou égale à 1.5 fois, de préférence 1 fois la plus petite dimension de sa base, mesurée orthogonalement à ladite longueur.

3. Ensemble selon la revendication 1 ou 2, **caractérisé en ce que** la canule et le capuchon comportent une section transversale généralement circulaire.

4. Ensemble selon l'une quelconque des revendications précédentes, **caractérisé en ce que** la longueur de ladite partie inférieure (2) du capuchon est inférieure ou égale à 1 cm.

5. Ensemble selon l'une quelconque des revendications précédentes, **caractérisé en ce que** les parties inférieure (2) et supérieure (3) du capuchon font corps l'une avec l'autre et **en ce qu'**une entaille périphérique (4) est agencée entre elles pour permettre de détacher la partie supérieure (3) de l'inférieure (2) par l'application de ladite flexion sur la face latérale de ladite partie supérieure (3).

6. Ensemble selon l'une quelconque des revendications précédentes, **caractérisé en ce que** le sommet de la partie inférieure (2) dudit capuchon présente, une fois la partie supérieure (3) du capuchon détachée de sa partie inférieure (2), un rebord (5) agencé pour définir avec la face latérale de la canule un canal périphérique pour récolter d'éventuelles impuretés présentes sur une tétine une fois ladite canule introduite dans cette tétine.

7. Ensemble selon l'une quelconque des revendications précédentes, **caractérisé en ce que** ladite partie inférieure (2) est munie d'une lèvre périphérique (6) agencée pour coopérer par forçage avec une gorge correspondante agencée à la base de la canule pour assurer la fixation de ladite partie inférieure (2) du capuchon à ladite base de la canule.

8. Ensemble selon l'une quelconque des revendications précédentes, **caractérisé en ce que** le diamètre supérieur (d1) et le diamètre inférieur (d2) du capuchon sont soit égaux, soit le diamètre inférieur est supérieur au diamètre supérieur.

## Claims

1. Assembly comprising a cowl (1) and an intra-mammary injection syringe provided with a canula and a reservoir and arranged to inject a medicinal substance into the channel of a teat of the udder of an animal, the said canula and the cowl being manufactured from an elastic material, the said cowl comprising a bottom part (2) arranged so as to be fixed to the base of the canula at a height where the canula is fixed to the reservoir, the said cowl also comprising a top part (3) secured to the bottom part (2) by a connection and arranged to cover the top part of the canula, the bottom (2) and top (3) parts of the cowl (1) being arranged so that, when it is fixed to the canula, the top part (3) of the cowl is detachable from its bottom part (2) by a movement exerted on the top part of the cowl without the bottom part (2) being thus detached from the canula, the said connection between the bottom part and the top part of the cowl being arranged so as to be broken under the effect of the said movement formed by the application of a flexion causing a lateral inclination of the said top part (3), **characterised in that** the bottom part (2) of the cowl has a length, considered in its axial direction, that is less than that of the top part (3).

2. Assembly according to claim 1, **characterised in that** the length of the said bottom part (2) of the cowl is less than or equal to 1.5 times and preferably the same as the smallest dimension of its base, measured orthogonally to the said length.

3. Assembly according to claim 1 or 2, **characterised in that** the canula and the cowl have a roughly circular transverse section.

4. Assembly according to any one of the preceding claims, **characterised in that** the length of the said bottom part (2) of the cowl is less than or equal to 1 cm.

5. Assembly according to any one of the preceding claims, **characterised in that** the bottom (2) and top (3) parts of the cowl are in a single piece and **in that** a peripheral notch (4) is arranged between them to make it possible to detach the top part (3) from the bottom one (2) by application of the said flexion to the lateral face of the said top part (3).

6. Assembly according to any one of the preceding claims, **characterised in that** the top of the bottom part (2) of the said cowl has, once the top part (3) of the cowl is detached from its bottom part (2), a rim (5) arranged to define, with the lateral face of the canula, a peripheral channel for collecting any impurities present on a teat once the said canula is introduced into this teat.

7. Assembly according to any one of the preceding claims, **characterised in that** the said bottom part (2) is provided with a peripheral lip (6) arranged to cooperate by forcing with a corresponding groove arranged at the base of the canula in order to ensure the fixing of the said bottom part (2) of the cowl to the said base of the canula.

8. Assembly according to any one of the preceding claims, **characterised in that** the top diameter (d1) and the bottom diameter (d2) of the cowl are either equal or the bottom diameter is greater than the top diameter.

## Patentansprüche

1. Baugruppe, die eine Kappe (1) und eine intramammäre Injektionsspritze versehen mit einer Kanüle und einem Behälter aufweist, und die eingerichtet ist, um eine medikamentöse Substanz in den Kanal einer Zitze des Euters eines Tiers einzuspritzen, wobei die Kanüle und die Kappe aus einem elastischen Material hergestellt sind, wobei die Kappe einen unteren Teil (2) aufweist, der eingerichtet ist, um an der Basis der Kanüle in der Höhe eingerichtet zu werden, in der die Kanüle an dem Behälter befestigt ist, wobei die Kappe auch einen oberen Teil (3) aufweist, der mit dem unteren Teil (2) durch eine Verbindung fest verbunden und eingerichtet ist, um den oberen Teil der Kanüle abzudecken, wobei der untere Teil (2) und der obere Teil (3) der Kappe (1) derart eingerichtet sind, dass der obere Teil (3) der Kappe, wenn sie an der Kanüle befestigt ist, von seinem unteren Teil (2) durch eine Bewegung abnehmbar ist, die auf den oberen Teil der Kappe ausgeübt wird, ohne dass der untere Teil (2) dabei von der Kanüle getrennt wird, wobei die Verbindung zwischen dem unteren Teil und dem oberen Teil der Kappe eingerichtet ist, um unter der Einwirkung der Bewegung, die aus Anlegen einer Biegung, die eine seitliche Neigung des oberen Teils (3) verursacht, besteht, gelöst zu werden, **dadurch gekennzeichnet, dass** der untere Teil (2) der Kappe eine Länge in seine axiale Richtung betrachtet aufweist, die kleiner ist als die des oberen Teils (3).

2. Baugruppe nach Anspruch 1, **dadurch gekennzeichnet, dass** die Länge des unteren Teils (2) der Kappe kleiner oder gleich 1,5 Mal, vorzugsweise 1 Mal so groß ist wie das kleinste Maß seiner Basis, orthogonal zu der Länge gemessen.

3. Baugruppe nach Anspruch 1 oder 2, **dadurch gekennzeichnet, dass** die Kanüle und die Kappe einen im Allgemeinen kreisförmigen Querschnitt aufweisen.

4. Baugruppe nach einem der vorhergehenden Ansprüche, **dadurch gekennzeichnet, dass** die Länge des unteren Teils (2) der Kappe kleiner oder gleich 1 cm ist.

5. Baugruppe nach einem der vorhergehenden Ansprüche, **dadurch gekennzeichnet, dass** der untere Teil (2) und der obere Teil (3) der Kappe miteinander eine Einheit bilden und dass eine periphere Einkerbung (4) zwischen ihnen eingerichtet ist, um es zu erlauben, den oberen Teil (3) von dem unteren Teil (2) durch Anlegen der Biegung auf der seitlichen Fläche des oberen Teils (3) zu trennen.

6. Baugruppe nach einem der vorhergehenden Ansprüche, **dadurch gekennzeichnet, dass** der Scheitel des unteren Teils (2) der Kappe, sobald der obere Teil (3) der Kappe von ihrem unteren Teil (2) getrennt ist, einen Rand (5) aufweist, der eingerichtet ist, um mit der seitlichen Fläche der Kanüle einen peripheren Kanal zu definieren, um eventuelle Verunreinigungen zu sammeln, die auf einer Zitze gegenwärtig sind, sobald die Kanüle in diese Zitze eingefügt ist.

7. Baugruppe nach einem der vorhergehenden Ansprüche, **dadurch gekennzeichnet, dass** der untere Teil (2) mit einer Umfangslippe (6) versehen ist, die eingerichtet ist, um durch Forcieren mit einer entsprechenden Hohlkehle, die an der Basis der Kanüle eingerichtet ist, zusammenzuwirken, um das Befestigen des unteren Teils (2) der Kappe an der Basis der Kanüle sicherzustellen.

8. Baugruppe nach einem der vorhergehenden Ansprüche, **dadurch gekennzeichnet, dass** der obere Durchmesser (d1) und der untere Durchmesser (d2) der Kappe gleich sind, oder dass der untere Durchmesser größer ist als der obere Durchmesser.
